# EUROPÄISCHE PATENTANMELDUNG

(11) **EP 0 994 193 A1**
(43) Veröffentlichungstag der Anmeldung: **19.04.2000**
(21) Anmeldenummer: 99890271.2
(22) Anmeldetag: 23.08.1999
(51) Int. Cl.: C12Q 1/68, G01N 27/447, G06F 19/00

(54) **Verfahren zum Nachweis von Organismen anhand ihrer Nukleinsäure**

(30) Priorität: 31.08.1998 DE 19839573
(71) Anmelder: Rölleke, Sabine, Dr., 1130 Wien (AT); Krech, Ralph, 1130 Wien (AT)
(72) Erfinder: Rölleke, Sabine, Dr., 1130 Wien (AT); Krech, Ralph, 1130 Wien (AT)

(57) **Zusammenfassung**

Die Erfindung betrifft ein Verfahren zum Nachweis von Organismen anhand von Nukleinsäureabschnitten ihrer DNA, sowie eine Vorrichtung zur Durchführung des erfindungsgemässen Verfahrens.

## Beschreibung

Die Erfindung betrifft ein Verfahren zum Nachweis von Organismen anhand von Nukleinsäureabschnitten ihrer DNA, sowie eine Vorrichtung zur Durchführung des erfindungsgemässen Verfahrens.

### 1. Herkömmliche Verfahren

### 1.1. Klassische Verfahren

Klassische mikrobiologische Verfahren zum Nachweis von Organismen in Proben beruhen auf einer Kultivierung der Keime und anschließender Bestimmung auf speziellen Nährmedien, die für bestimmte Organismen spezifisch sind. Mit diesen Verfahren ist jedoch ein Zeitaufwand von mehreren Tagen verbunden.

### 1.2. Nukleinsäure-Nachweisverfahren

Eine schnellere Bestimmung kann durch Nukleinsäure-Nachweisverfahren erfolgen.

Bei herkömmlichen DNA-analytischen Nachweisverfahren handelt es sich um PCR Testsysteme, die gegenüber den oben genannten klassischen Methoden einen schnelleren und sensitiveren Nachweis von Keimen in Proben erlauben. Spezifische PCR Testsysteme weisen jeweils einen bestimmten Keim oder eine Gruppe von Keimen (ohne weitere Spezifizierungsmöglichkeit) nach (z.B. Salmonellen auf Hühnchen, Borrelien in Blut, eine gentechnisch veränderte Starterkultur in Joghurt, Babesien in Katzenblut). Eine besondere und neuere Form eines PCR Testsystems ist die sogenannte "Multiplex-PCR" . Sie basiert auf der Verwendung von mehreren spezifischen Primerpaaren in einem einzigen Reaktionsansatz (z.B. Nachweis von Borrelien, FSME und Rickettsien in einer Reaktion). Gemeinsam ist diesen Nachweissystemen (auch der "Multiplex-PCR"),
a) daß sie für jeden nachzuweisenden Keim entwickelt werden müssen. Dies ist zeitaufwendig und teuer, und
b) es werden nur solche Keime nachgewiesen, nach denen auch gesucht wird. Beispielsweise kann mit einem spezifischen PCR Test auf Borrelien kein anderer Keim identifiziert werden. Dies hat zur Folge, daß häufig pathogene (Mikro-)Organismen (etwa in Lebensmitteln oder klinischen Proben) nicht gefunden werden.
2. SequenzsDezifische Auftrennung von Nukleinsäuren

### 2.1. Gele mit Denaturierungsgradienten

Diese Verfahren beruhen darauf, daß Sequenzvariationen zwischen verschiedenen DNA Molekülen zu einem veränderten Schmelzverhalten der Doppelstränge führen. Diese unterschiedlich stark geschmolzenen Doppelstränge besitzen trotz gleicher Länge eine unterschiedliche Mobilität in einer Gelmatrix. Dies ist darauf zurückzuführen, daß sich sogenannte Schmelzdomänen ausbilden, d.h. Abschnitte innerhalb des DNA Fragments, in denen die DNA denaturiert. Verfahren, die auf diesem Prinzip beruhen, sind die DGGE (Denaturing Gradient Gel Electrophoresis, Denaturierungsgradienten-Gelelektrophorese) und TGGE (Temperature Gradient Gel Electrophoresis, Temperaturgradienten-Gelelektrophorese), die z.B. Millar et al. (J.Clinical Microbiol., 34, 2506-2510 (1996), Muyzer et al. (Appl.Envir.Microbiol., 59, 695-700 (1993) und Rölleke et al. (Appl.Envir.Microbiol., 62, 2059-2065 (1996) beschrieben sind. Dort werden die DNA-Fragmente auf Acrylamid-Gelen aufgetrennt, welche eine ansteigende Konzentration an Denaturierungsmitteln aufweisen. Bei einer Konzentration, die für jede Sequenz spezifisch ist, wird die DNA denaturiert, was sich in einer stark verlangsamten Migrationsgeschwindigkeit oder Stillstand der Banden bemerkbar macht.

Zur Optimierung der Auftrennung von DNA-Fragmenten in der DGGE/TGGE wird bisher im allgemeinen eine GC-Klammer (GC reiche Region, die in der Regel ca. 40 Basen lang ist) über die PCR an die aufzutrennenden Fragmente gehängt. Damit soll verhindert werden, daß sich einzelne Nukleinsäure-Stränge gänzlich voneinander trennen. In bisherigen manuellen DGGE/TGGE Verfahren konnte mit dieser Klammer eine erheblich bessere Auftrennung der Fragmente erreicht werden, weil die Fragmente fast völlig zum Stillstand kommen, distinkte Banden formen und deshalb manuell aus dem Gel ausgeschnitten werden können. Eine direkte Identifizierung von Organismen anhand der Migrationswerte ihrer Nukleinsäureabschnitte und/oder eine Online-Detektion während oder nach der Migration ist mit einer solchen Technologie allerdings nicht möglich.

Bisher mußte immer das gewünschte DNA-Fragment durch Ausschneiden des betreffenden Gelstücks aus dem Gel isoliert werden, was wiederum aufwendige Arbeitsschritte mit sich bringt. Zudem ist eine Elution aus ausgeschnittenen Gelstücken immer mit einem großen Verlust im Nukleinsäurematerial verbunden.

Die Anwendung der DGGE zum Nachweis von Mikroorganismen ist bereits für durch Mikroorganismen geschädigte Wandgemälde (Fresken) beschrieben worden (Rölleke et al., Appl. Environm. Microbiol., 62, 2059-2065 (1996). Auch in der medizinischen Diagnostik wurde dieses Verfahren zur Untersuchung von Säuglingsfäkalienproben bereits verwendet (Millar et al., J. Clin. Mikrobiol. 34, 2506-2510 (1996). Allerdings wurde eine automatisierte Detektion mit anschließender Identifikation der Organismen bisher noch nicht vorgeschlagen.

### 2.2 Sequenzspezifische DNA Liganden

Gleich große, aber in der Sequenz verschiedene DNA Moleküle können durch DNA Liganden, die an bestimmte Sequenzabfolgen binden, voneinander getrennt werden. Diese Trennung beruht darauf, daß die spezifischen kurzen Sequenzabfolgen, an die der DNA-Ligand bindet, in DNA-Molekülen mit unterschiedlicher Sequenz in unterschiedlicher Anzahl auftreten, so daß unterschiedliche Anzahlen der DNA-Liganden an ein bestimmtes DNA Molekül binden. Dies führt dann zu einer Änderung des Gewichtes und der Größe des DNA-Ligand-Komplexes, wodurch dann eine sequenzspezifische Auftrennung ermöglicht wird. Ein Beispiel für einen spezifischen DNA-Liganden ist das Bisbenzimid-PEG-Konjugat, Handelsname: H.A Yellow, was unter anderem bei Müller et al. (NAR, 25, 5125-5126 (1997) beschrieben ist. Die Detektion erfolgte durch Ethidium-Bromid-Färbung der Agarosegele nach der Elektrophorese und anschließendes Fotografieren unter UV Licht. Ein Hinweis auf eine automatisierte Detektion findet sich nicht. Ein Nachteil dieses Verfahrens ist allerdings, daß es nicht zwischen allen möglichen Sequenzvariationen unterscheidet. Bei H.A. Yellow verursacht z.B. CAC → CGC keine Mobilitätsveränderung.

### 2.3. rSSCP-DNA-SSCP (RNA/DNA-Einzelstrang-Konformationspolymorphismus)

Einzelsträngige Nukleinsäuren bilden unter nicht-denaturierenden Bedingungen eine Sekundärstruktur aus, d.h. es entstehen doppelsträngige Bereiche. Unterschiedliche Sequenzen bilden unterschiedliche Sekundärstrukturen, woraus sich wie bei der DGGE ein unterschiedliches Laufverhalten für gleich lange Fragmente mit unterschiedlicher Sequenz ergibt. Im Stand der Technik ist SSCP im Vergleich mit DNA-SSCP beispielsweise durch Sarka et al. (NAR, 20, 871-878) (1992)) beschrieben. Zur Detektion wurden die Nukleinsäuren vor der Auftrennung auf einen Polyacrylamidgel markiert und das Gel unter UV Licht fotografiert. Auch hier würde eine automatisierte Detektion eine große Zeitersparnis bedeuten.

### 3. Gegenstand der Erfindung

An das Analyseverfahren der klinischen Diagnostik, z.B. auf dem Gebiet der Parasitologie, und der Lebensmittelanalytik werden zunehmend höhere Anforderungen gestellt. Hierbei ist vor allen Dingen die Zeit ein wichtiger Faktor. Beispielsweise ist der Nachweis von relevanten Organismen, z.B. Mikroorganismen wie Bakterien, Pilze, Hefen und andere Einzeller, aber auch höhere Organismen, wie etwa Parasiten, Würmer oder auch Pflanzen, Säugetiere und Menschen, sehr zeitaufwendig und oftmals sogar unmöglich. Dies beruht auf dem Mangel an automatisierten Nachweisverfahren, welche eine Probenanalyse bei geringem Zeitaufwand und mit geringen Kosten und wenigen Arbeitsschritten ermöglichen.

Wie bereist erwähnt, müssen in bekannten Nukleinsäure-Nachweisverfahren die entsprechenden Banden aus dem Gel ausgeschnitten und anschließend entweder wiederum amplifiziert und sequenziert werden. Die Sequenzen werden dann mit Sequenzen von bekannten (Mikro-)Organismen verglichen und so bestimmten (Mikro-)Organismen zugeordnet.

Der Erfindung lag daher die Aufgabe zugrunde, ein Verfahren zum Nachweis von Organismen anhand von Nukleinsäureabschnitten ihrer DNA bereitzustellen, welches für eine schnelle und kostengünstige Analyse von DNA Proben geeignet ist.

Die Erfindung erlaubt die Identifikation von in einer Ausgangsprobe enthaltenen Organismen aufgrund des Migrationsverhaltens ihrer spezifischen Nukleinsäureabschnitte
(a) ohne Notwendigkeit der Sequenzierung von Nukleinsäureabschnitten nach der Auftrennung durch die Migration durch eine Matrix, wenn die Sequenz des spezifischen Nukleinsäureabschnitts eines Organismus und sein Migrationsverhalten bekannt ist, und
(b) wenn die Sequenz des spezifischen Nukleinsäureabschnitts eines Organismus und sein Migrationsverhalten nicht bekannt ist, durch Eluierung (Sammeln) der Nukleinsäureabschnitte zur anschließenden Sequenzierung.

Bekannt heißt hier, daß ihre Sequenz und ihr Migrationsverhalten unter standardisierten Bedingungen bekannt ist, beispielsweise
(a) durch die Zeit, die der Nukleinsäureabschnitt des Organismus benötigt, um durch die Matrix zu migrieren oder
(b) durch die bestimmte Position der aufgetrennten Sequenzen zu einer bekannten Sequenz in einer Matrix.

Die Aufgabe wird erfindungsgemäss gelöst durch ein Verfahren zum Nachweis von Organismen anhand ihrer Nukleinsäure, umfassend die Schritte:
(a) Bereitstellen einer Probe, die einen Organismus oder verschiedene Organismen oder genetisches Material davon enthalten kann,
(b) Organismusübergreifendes Erzeugen von Nukleinsäureabschnitten der Organismen, die eine sequenzspezifische Zuordnung zu den entsprechenden Organismen erlauben,
(c) Sequenzspezifisches Auftrennen der Nukleinsäureabschnitte mittels Migration durch eine Matrix,
(d) Automatisiertes Detektieren der aufgetrennten Nukleinsäureabschnitte während und/oder nach der Migration durch einen stationären oder mobilen Detektor,
(e) Mindestens teilweises Identifizieren von Organismen aufgrund der sequenzspezifischen Mobilität ihrer Nukleinsäureabschnitte durch Vergleich mit in einer Datenbank gespeicherten Sequenzen bekannter Organismen und der Mobilitätswerte dieser Sequenzen.

In diagnostischen und analytischen Nachweisverfahren kann diese Probe beispielsweise Wasser, Abwasser oder eine sonstige Flüssigkeit, z.B. eine Körperflüssigkeit sein, wie etwa Blut, Urin, Speichel, Kot und dergleichen, sowie Gewebe- und Zellproben. In der Lebensmittelanalytik können jedwede Lebensmittelproben, wie etwa Fleisch, Milchprodukte, u.ä. untersucht werden. Außerdem können mit dem hier vorgeschlagenen Verfahren auch Werkstoffe, wie etwa Baumaterial, sowie Kunstobjekte oder Antiquitäten untersucht werden.

Die zu untersuchenden Nukleinsäuren werden durch herkömmliche Verfahren extrahiert. Anschließend werden sie gespalten oder vervielfältigt, aufgetrennt und detektiert. Bevorzugt werden die Nukleinsäureabschnitte durch eine Vervielfältigung erzeugt. Die Vervielfältigung besteht in einem organismusübergreifenden Amplifizieren von Nukleinsäureabschnitten der zu untersuchenden biologischen Organismen. Bevorzugt wird die organismusübergreifende Amplifikation als PCR durchgeführt. Andere Amplifikationsformate sind jedoch ebenfalls denkbar. Beispiele hierfür sind die im Stand der Technik bekannten und dem Fachmann geläufigen NABA- oder LCR Verfahren. Zu amplifizierende Zielsequenzen werden aus innerhalb der definierten Organismengruppe hochkonservierten Sequenzen ausgewählt, wobei die Organismen aus beliebigen Organismen, also Prokaryonten, Eukaryonten und Archaea, ausgewählt werden können. Als geeignete Zielsequenz haben sich für ribosomale RNA codierende DNA-Sequenzen, erwiesen, z.B., beim Nachweis von Bakterien die für 16S rRNA codierenden DNA Sequenzen. Derartige Amplifikationsverfahren sind beispielsweise bei Medlin et al. Gene 71, 491-499 (1988), und Muyzer et al. (supra) beschrieben. Bei der (PCR)-Amplifikation ist zu amplifizierende Nukleinsäure bevorzugt DNA oder RNA, und die Nukleinsäureabschnitte (Amplifikationsprodukte) sind bevorzugt DNA.

Es ist jedoch auch denkbar, die zu unterscheidenden Nukleinsäureabschnitte auf andere Art und Weise zu erzeugen, beispielsweise durch Klonierung oder andere Arten der Nukleinsäuresynthese. Die zu untersuchenden Nukleinsäuren können auch z.B. chemisch, physikalisch oder enzymatisch in Abschnitte gespalten werden (spezifische Fragmentierung).

Ein geeigneter PCR Primer für die Detektion von Bakterien ist beispielsweise bei Rölleke et al. (supra) beschrieben, so daß hier nicht näher darauf eingegangen werden muß. Die organismusübergreifend amplifizierten DNA Fragmente haben vorzugsweise eine Länge zwischen 100 und 2000 bp, können jedoch auch erheblich größer oder kleiner sein. Bevorzugt ist eine Länge um 200bp, wie bei Rölleke et al. beschrieben. Derzeit bevorzugt handelt es sich für Bakterien um 16S rDNA amplifizierende Primer, wobei das Verfahren selbstverständlich auch für die Untersuchung von Abschnitten aus anderen Genen verwendbar ist. Das erfindungsgemässe Verfahren kann nicht nur spezies- oder organismusspezifische Amplifikationsprodukte nachweisen, sondern es ermöglicht auch überraschenderweise bei der Auswahl von geeigneten Primern den Nachweis von genetisch (gentechnologisch) veränderten Organismen. Dieser Aspekt ist insbesondere in der Lebensmittelanalytik von Bedeutung, da Nahrungsmittel in zunehmenden Masse gentechnisch veränderte Pflanzenbestandteile oder andere Organismen (beispielsweise Joghurtkulturen) enthalten. Die zu untersuchenden Nukleinsäurefragmente können während oder nach der Amplifizierung gegebenenfalls auch markiert werden, beispielsweise durch den Einbau von radioaktiven Nukleotiden. Vorzugsweise sind die markierenden Gruppen jedoch nicht radioaktiv, sondern basieren beispielsweise auf fluoreszierenden Molekülen.

Organismusübergreifend bedeutet bei einem Erzeugen von Nukleinsäureabschnitten durch Amplifikation, daß beispielsweise Nukleinsäuren einer definierten Gruppe selektiv amplifiziert werden können. Da für das erfindungsgemässe Verfahren bevorzugt Nukleinsäureabschnitte mit gleicher Länge aber unterschiedlicher Sequenz verglichen werden, bedeutet organismusübergreifend zum einen, daß die Nukleinsäureabschnitte aus entsprechenden Regionen der unterschiedlichen Genome der zu untersuchenden Organismen erzeugt werden (z.B. aus demselben Gen). Damit ist z.B. ein genus-, gattungs-, oder auch speziesübergreifender Nachweis möglich. Andererseits können aber auch individuen-spezifische Nukleinsäuren unterschieden werden. Dies ist besonders bei der Erstellung und Untersuchung von genetischen Fingerabdrücken von Bedeutung. Es können aber auch Polymorphismen nachgewiesen werden, indem Nukleinsäureabschnitte aus polymorphen Regionen des Genoms verwendet werden.

Durch das erfindungsgemässe Verfahren werden die für _{"}herkömmliche" Nukleinsäure-Nachweisverfahren vorhandenen Einschränkungen überwunden. So erlaubt die Verwendung der organismusübergreifenden Primer, die an zwischen einzelnen Mitgliedern der Gruppe hochkonservierte Bereiche binden, den Nachweis aller Gruppenmitglieder, ohne sie vorher zu erkennen oder nach ihnen spezifisch zu suchen. Durch entsprechende Primerwahl können beispielsweise (Mikro-)Organismen einer bestimmten definierten Gruppe, z.B. alle Bakterien oder alle Pilze oder alle Eukaryonten oder alle gram-positiven Bakterien, alle Spezies des Genus Salmonella etc. nachgewiesen werden.

Ein besonderer Vorteil dieses Ansatzes besteht darin, daß bei Verwendung von Referenzen als Standard - wie weiter unten ausführlich erläutert - auf beliebig viele Organismen gleichzeitig getestet werden kann. Weiterhin können auch Organismen aufgefunden werden, die nicht im Standard enthalten sind, beispielsweise weil sie vorher gar nicht oder noch nie in einem bestimmten Probenmaterial detektiert wurden. Dieses Auffinden neuer Organismen kann durch Sequenzieren von nicht den Referenzen entsprechenden Banden erfolgen. Dieser Vorteil ist besonders deshalb wichtig, weil nach herkömmlichen Methoden nur diejenigen Sequenzen gefunden werden können, nach denen gezielt gesucht wird.

Darüber hinaus erlaubt das erfindungsgemässe Verfahren in vielen Fällen auch die Detektion genetisch veränderter Organismen.

Zum sequenzspezifischen Auftrennen werden die in Schritt (b) erhaltenen Nukleinsäureabschnitte in eine Matrix eingebracht. Die für das erfindungsgemässe Verfahren geeignete Matrix erlaubt das sequenzspezifische Auftrennen von Nukleinsäureabschnitten in räumlich voneinander getrennte Banden. Bevorzugt ist diese Matrix eine Gelmatrix, wie etwa ein Agarose- oder Polyacrylamidgel, und die Migration durch die Matrix erfolgt bevorzugt durch Elektrophorese. Erfindungsgemäss ist es auch denkbar, als Matrix eine Flüssigkeit oder einen festen Stoff zu verwenden, beispielsweise für eine Auftrennung über Zentrifugation oder Chromatographie / Massenspektoskopie.

Die zu untersuchenden Nukleinsäureproben werden vor dem Einbringen in die Matrix bevorzugt in einer bestimmten Menge Wasser oder Puffer gelöst. Der Trennmatrix kann bevorzugt eine Sammelmatrix, z.B. ein Sammelgel, vorgeschaltet sein. Durch die Sammelmatrix wird eine verbesserte Detektion auch geringer Unterschiede ermöglicht, da die Nukleinsäurefragmente während ihrer Wanderung durch die Sammelmatrix auf einen sehr begrenzten Bereich komprimiert werden, so daß am Beginn der Trennmatrix ein einheitlicher Migrationsanfangspunkt vorliegt. Als Sammelmatrix wird z.B. ein nichtdenaturierendes Gel mit geringerer Konzentration als das Trenngel verwendet.

In Schritt (c) des erfindungsgemässen Verfahrens wird ein sequenzspezifisches Auftrennen der Nukleinsäureabschnitte oder Amplifikationsprodukte in Fraktionen mit unterschiedlicher Mobilität durchgeführt. Diese Auftrennung kann nach einem der bereits genannten Verfahren durchgeführt werden: DGGE (Denaturierungsgradienten-Gelelektophorese), TGGE (Temperaturgradienten-Gelelektrophorese), sequenzspezifische Bindung an DNA-Liganden, rSSCP (RNA-Einzelstrang-Konformationspolymorphismus), DNA-SSCP (DNA-Einzelstrang-Konformationspolymorphismus) oder nach jedem anderen Verfahren, das eine sequenzspezifische Auftrennung von Nukleinsäuren ermöglicht. Bevorzugt ist für das erfindungsgemässe Verfahren die DGGE bzw. die TGGE. Die erfindungsgemäß verwendbaren Verfahren umfassen natürlich auch Abwandlungen der oben genannten Auftrennungsverfahren, wie sie z.B. durch Veränderung einzelner Parameter, wie etwa der Puffer- oder Denaturierungsmittelkonzentrationen, entwickelt werden können.

Bei der Migration durch eine Matrix wandern manche Nukleinsäuremoleküle schneller als andere, was dann eine Auftrennung von Fraktionen, die gleichartige Nukleinsäurefragmente enthalten, in sogenannte Banden zur Folge hat. Im Stand der Technik wurden diese Banden immer manuell und stehend detektiert, z.B. als Fotografie unter UV Licht oder als Autoradiographie.

Wie oben bereits erwähnt, werden bei im Stand der Technik bekannten TGGE- oder DGGE-Auftrennungsverfahren üblicherweise GC-Klammern eingesetzt mit dem ebenfalls bereits erläuterten Nachteil, daß es dadurch zu einem Stillstand der Banden auf der Matrix kommt und eine Online-Detektion nicht möglich ist. Gleichzeitig würde aber das Weglassen einer solchen Klammer unter Beibehalten der bisherigen Auftrennungsmethode dazu führen, daß die Banden nicht mehr distinkt voneinander getrennt werden könnten, sondern sich Einzelstränge abtrennen würden, die nicht mehr detektiert werden könnten. Deshalb umfaßt die vorliegende Erfindung auch eine völlige technische Veränderung der bislang bekannten Gradiententechnologe. Dies kann wie folgt geschehen:

### a) Modifizierung des Gradienten

Die vorliegende Erfindung verwendet bevorzugt einen chemischen Stoff (beispielsweise Harnstoffe oder dergleichen) zur Denaturierung der Wasserstoffbrückenbindungen zwischen den Basen-Komplementären. Die Konzentration dieses Stoffes in der Matrix variiert in definierter Weise bis zu einem Punkt, in dem die Nukleinsäure-Sequenzen zwar je nach ihrer Zusammensetzung früher oder später voneinander getrennt werden (Schmelzpunkt), jedoch durch die Spaltung der Wasserstoffbrücken nicht gänzlich zum Stehen kommen, sondern weiter durch die Matrix migrieren. Das gleiche Ergebnis kann auch durch eine definierte Abfolge von Temperaturveränderungen erfolgen oder durch die Verwendung eines sonstigen chemischen oder physikalischen Vorgangs, mit dessen Hilfe Wasserstoffbrücken zwischen den Basen-Komplementären kontrolliert gespalten oder eine solche Spaltung gestoppt werden können. Dieses System kann erfindungsgemäß jedoch auch gekoppelt werden mit einem Anhängen eines in ihrer Anzahl und Zusammensetzung so gewählten GC-Komponente, daß sich die Nukleinsäure-Stränge nicht gänzlich voneinander trennen.

### b) Modifizierung der Elektophoresebedingungen

Eine Detektion während der Migration ist auch möglich, indem eine Abfolge definierter Stromspannungsveränderungen während der Migrationsphase erfolgt. Dabei wird bevorzugt zum Ende der Migrationsphase die Stromspannung erheblich erhöht werden, um zu gewährleisten, daß auch solche Fragmente, deren Wasserstoffbrücken fast vollständig aufgetrennt wurden, nicht gänzlich zum Stillstand kommen.

Gleichfalls ist das Stehenbleiben der zu detektierenden Banden auch durch eine Abfolge definierter Veränderungen der Orientierung des Stromspannungsfeldes möglich.

Die vorliegende Erfindung demonstriert, daß überraschenderweise auch für die oben genannten Auftrennungsverfahren eine automatisierte Detektion, d.h. eine Detektion an die sich eine Identifizierung direkt anschließt, vorteilhaft eingesetzt werden kann. Bevorzugt handelt es sich um eine Online-Detektion, bei der der Detektor mit einer Steuereinheit verbunden ist, welche die Identifizierung der Banden erlaubt. Dabei werden die Banden vorzugsweise durch einen nahe dem Ende der Matrix plazierten und im allgemeinen stationären Detektor erfaßt. Da dies zu unterschiedlichen Zeitpunkten ab dem Zeitpunkt des Beginns der Migration geschieht, können den einzelnen Banden jeweils unterschiedliche Werte für die Mobilität zugeordnet werden. Diese ergibt sich aus dem in einer bestimmten Zeit und unter bestimmten Bedingungen (wie etwa der Pufferkonzentration, Gelkonzentration, Temperatur, Spannung und dergleichen) zurückgelegten Weg ab dem Punkt des Migrationsbeginns bis zum Detektor. Besonders bevorzugt erfolgt die Detektion während der Wanderung der Banden durch die Matrix, sie kann aber auch anschließend erfolgen.

Alternativ können jedoch auch andere automatisierte Detektionsmöglichkeiten verwendet werden. So können nach sequenzspezifischer Auftrennung, bei der die Fragmente innerhalb der Matrix zum Stehen kommen, wie üblicherweise bei bekannten DGGE- oder TGGE-Verfahren, ein digitales Bild des Bandenmusters erstellt werden, indem ein mobiler Detektor verwendet wird, der z.B. nach der Auftrennung an der Matrix entlangfährt und die Mobilitätswerte der augetrennten Nukleinsäurefragmente mit den Mobilitätswerten der angeschlossenen Datenbank vergleicht und, wenn ein entsprechender Mobilitätswert in der Datenbank nicht vorhanden ist, gegebenenfalls veranlaßt, daß die gewünschten Banden automatisch aus der Matrix abgesogen werden. Dies könnte durch Kombination der Matrix mit einem Vakuumblotsystem erfolgen, welches in der Lage ist, ein punktgenaues Vakuum an die Matrix anzulegen, wodurch spezifische Fragmente selektiv abgesogen und in einem Behälter aufgefangen werden können.

Da die Mobilität für Nukleinsäurefragmente gleicher Länge sequenzspezifisch ist, können die Nukleinsäureabschnitte oder Amplifikationsprodukte aufgrund ihrer Mobilität identifiziert werden.

Identifizieren heißt die Zuordnung eines unbekannten Organismus andhand von Nukleinsäureabschnitten zu einem bekannten Organismus oder einer Gruppe von Organismen. Gruppen von Organismen können auf allen phylogenetischen Ebenen definiert werden. Eine Gruppe kann auch Organismen mit definierten Eigenschaften umfassen (z.B. alle nitrifizierenden Mikroorganismen).

Eine Identifizierung kann auch anhand einer phylogenetischen Zuordnung erfolgen, d.h., wenn die Nukleinsäuren nicht zu 100 %, sondern zu einem geringeren Prozentsatz mit dem bekannten Organismus identisch sind. Dies wird von der Datenbank entsprechend abgeglichen und angezeigt, insbesondere wo die Sequenzabweichungen auftreten.

Die Identifizierung kann auf zwei Arten erfolgen.

In einer ersten Ausführungsform migrieren gleichzeitig mit der Probe bekannte Standards durch die Matrix mit. Diese Standards enthalten Nukleinsäureabschnitte, z.B. Amplifikationsprodukte von Nukleinsäuren bekannter Organismen, die wie auch die Probe durch die Matrix aufgetrennt werden. Durch den Vergleich der Mobilitäten der Nukleinsäureabschnitte aus der Probe mit den Nukleinsäureabschnitten aus dem Standard wird eine Identifizierung ermöglicht.

In einer zweiten, bevorzugten, Ausführungsform ist ein Detektor an eine Steuereinheit angeschlossen, welche unter anderem eine Datenbank mit gespeicherten Informationen zu Mobilitäten bekannter Sequenzen enthält. Diese Daten wurden ermittelt, indem Nukleinsäureabschnitte bekannter Organismen unter den gleichen Bedingungen durch die erfindungsgemäße Matrix migrierten, wie diejenigen, unter denen die zu untersuchenden Proben durch die Matrix migrieren. Nun können die vom Detektor ermittelten Mobilitätswerte mit zuvor gespeicherten Daten der Mobilität der Nukleinsäureabschnitte bekannter Organismen verglichen werden und die Identifizierung auf diese Weise erfolgen. Bei gleicher Migrationsdauer unter gleichen Migrationsbedingungen ist der durch die erfindungsgemäße Matrix migrierte Nukleinsäureabschnitt einem bekannten Organismus zuordnungsbar.

Um standardisierte Migrationsbedingungen zu gewährleisten, wird vorzugsweise vor dem Probenauftrag das Gerät geeicht. Dies kann beispielsweise dadurch erfolgen, indem ein Referenzstandard durch die Matrix migriert (wie z.B. bei der Eichung einer HPLC).

Je mehr Mobilitätswerte der Sequenzen bekannter Organismen in der Datenbank vorhanden sind, desto weniger Bedarf besteht an einer anschließenden Sequenzierung. Das bedeutet, daß letztlich die Sequenzierung, wie sie bislang regelmäßig nach der Auftrennung erfolgen muß, durch die Erfindung weitgehend ersetzt werden kann und nur noch dann erfolgen muß, wenn etwa in wenigen Fällen eine eindeutige Zuordnung nicht möglich sein sollte oder ein Organismus noch mit keinem Migrationswert seines Nukleinsäureabschnitts assoziiert ist.

Beiden Ausführungsformen ist gemein, daß sie auch so eingerichtet werden können, nur bestimmte Organismen anzuzeigen, auf die es in einer Probe ankommen mag (z.B. nur pathogene Organismen in einer Probe). Dies kann beispielsweise erfolgen, indem die Standards oder die Datenbank z.B. einen Standardset A und einen Standardset B umfasst, welche jeweils Abschnitte von Nukleinsäuren aus bekannten pathogenen und nichtpathogenen Organismen enthalten. Da das erfindungsgemässe Verfahren für lebensmittelanalytische und klinisch diagnostische Verfahren geeignet ist, werden bevorzugt Organismen, wie etwa Einzeller, z.B. Parasiten, Bakterien, Pilze und Hefen nachgewiesen. Bekannte pathogene Organismen, deren Nukleinsäurefragmente in Standard A enthalten sind, umfassen z.B. Salmonellen, Listerien, pathogene E.coli etc. Standardset B umfaßt beispielsweise in lebenden Joghurtkulturen anzutreffende Bakterien wie etwa Lactobacillus thermophilus. Es können auch Nukleinsäuren als Standards verwendet werden, die aus Organismen mit gentechnologisch verändertem Genom stammen. Diese können dann ebenfalls im Standardset A enthalten sein.

Weiterhin können die Standards auch Nukleinsäureabschnitte von Parasiten, z.B. Einzellern oder Würmern oder dergleichen enthalten. Prinzipiell sind Nukleinsäureabschnitte von allen obengenannten Organismen geeignet. Ebenso können auch Standards mit menschlichen Nukleinsäureabschnitten erstellt werden, z.B. zur Verwendung in der forensischen Medizin oder Kriminalistik.

Da bei einem solchen Verfahren und insbesondere unter Verwendung von bestimmten Standardsets nicht immer alle Banden identifiziert werden können, besteht zusätzlich die Möglichkeit, einzelne Banden mit aufgetrennten Nukleinsäureabschnitten zu sammeln. Dies ist möglich, da alle Banden beliebig lange durch die Matrix migrieren können und somit auch an einem bestimmten Zeitpunkt das Ende der Matrix erreichen. Sobald sie aus der Matrix austreten, können sie mit Hilfe von Kollektorbehältern aufgefangen werden. Im Gegensatz zu früheren Verfahren, wo immer bestimmte Banden aus dem Gel ausgeschnitten werden mußten und durch aufwendige Verfahren extrahiert werden mußten, können hier die Banden direkt aufgefangen werden, was zeit- und kostengünstiger ist.

Zudem werden Nukleinsäureabschnitte, anders als bei deren manueller Eluierung aus der Matrix, nicht mehr mit anderen zu dieser Bande nahegelegenen Nukleinsäuren kontaminiert. Die Banden können auch nicht mehr schlicht übersehen werden. Damit löst die Erfindung ein Problem, daß immer wieder kritisiert wird und für das viele Fachleute Lösungen suchen. Damit ist es auch nicht mehr notwendig, beispielsweise parallel aufwendige Klonierungen durchzuführen, in denen die durch DGGE oder TGGE produzierten Bandenmuster gescreent werden. Denn diese Herangehensweisen eignen sich nicht zur Routineuntersuchung, weil sie viel zu aufwendig sind.

Ein weiterer Aspekt der Erfindung ist, daß mit diesem Verfahren Nukleinsäuren verschiedener Organismen in einer Probe für lebensmittelanalytische oder parasitologische Zwecke nachgewiesen werden können.

Es können auch nur pathogene Organismen in einer Probe angezeigt werden.

Das Verfahren kann auch zum Nachweis von Individuen in der kriminalistischen Analytik verwendet werden.

Das Verfahren kann auch zum Nachweis von Mikroorganismen in und an Kunstgegenständen und Werkstoffen verwendet werden.

Das Verfahren kann auch zum Nachweis von Parasiten, z.B. Würmern und Einzellern, beispielsweise in biologischen Proben, verwendet werden.

Das Verfahren kann auch zum Nachweis in der Lebensmittel- und Abwasseranalytik verwendet werden.

Mit dem erfindungsgemäßen Verfahren können zusätzlich auch genetisch veränderte Organismen detektiert werden. Wie bereits erwähnt, ist dies von großer Bedeutung in der Lebensmittelanalytik, wenn z.B. genetisch veränderte Organismen, wie Pflanzen oder Tiere, identifiziert werden sollen, sofern noch genetisches Material in Nahrungsmitteln vorhanden ist. Die Identifizierung kann positiv erfolgen, indem bekannt genetisch veränderte Sequenzen bereits als Standard gespeichert sind oder als Kontrolle mitlaufen, oder negativ, wobei dann ein Sequenzierungsabschnitt erfolgen kann.

Das Verfahren kann auch zum Nachweis der Authentizität von Lebensmitteln (Erkennen von Lebensmittelvermischungen) verwendet werden.

Außerdem betrifft die Erfindung eine Vorrichtung zur Durchführung eines erfindungsgemässen Nachweisverfahrens. Diese Vorrichtung umfaßt:
(a) eine Matrix mit gegebenenfalls vorgeschalteter Sammelmatrix zum sequenzspezifischen Auftrennen in räumlich voneinander getrennte Banden von Nukleinsäureabschnitten mit annähernd gleicher Länge, wobei das Auftrennen mittels Migration der Nukleinsäureabschnitte vorzugsweise vollständig durch die Matrix hindurch geschieht,
(b) einen automatisierten Detektor zum Erfassen der aufgetrennten Nukleinsäureabschnitte während und/oder nach der Migration durch die Matrix.
(c) Gegebenenfalls einen Kollektor zum Sammeln der Fraktionen von Nukleinsäureabschnitten.

Die Matrix kann in verschiedenen Formen vorliegen, beispielsweise als Platte, Säule, Spiralsäule oder Spule, wodurch besonders lange Migrationswege erzielt werden. Die Matrix kann ein Gel sein, durch das die Migration bevorzugt durch Elektrophorese durchgeführt wird. Das Gel ist bevorzugt von einem Puffer umgeben, welcher bei Anlegen einer Spannung die Migration von Proben durch die Matrix erlaubt. Der Detektor zum Erfassen der aufgetrennten Nukleinsäureabschnitte mit bevorzugt gleicher oder ähnlicher Länge kann ein stationärer oder mobiler Detektor sein, wie sie beispielsweise im Rahmen automatischer Sequenzierung verwendet werden. Es kann auch jede andere Form der computergestützten Detektion der Banden verwendet werden.

Eine schematische Darstellung einer erfindungsgemäßen Ausführungsform der Vorrichtung ist in den Figuren 1 und 2 dargestellt. Dabei befindet sich ein Gel als Matrix (4) in einem Pufferbehälter (1), der mit Puffer (9) gefüllt ist. Der Gelmatrix (4) kann eine Sammelmatrix (2) vorgeschaltet sein, die Schlitze oder Taschen (3) für Proben aufweist. In dem Pufferbehälter (1) kann eine Spannung angelegt werden (5), wobei die Proben von dem Ende der Matrix, das die Taschen enthält, zum anderen Ende migrieren. Nahe dem Ende der Matrix befindet sich ein (IR)-Detektor (6), der mit einer Steuereinheit verbunden sein kann. Am Ende der Matrix befindet sich ein Kollektor (8), der ebenfalls mit der Steuereinheit (7) verbunden ist. In Figur 3 ist eine ähnliche Anordnung zu sehen, wobei die Matrix hier aus einer Spule besteht.

Der Detektor ist bevorzugt an eine Steuereinheit angeschlossen, umfassend (a) eine Datenbank zum Speichern von Werten für die Mobilität von detektierten Nukleinsäureabschnitten, (b) eine Vergleichs- und Zuordnungseinheit zum Vergleich der Mobilität von Nukleinsäureabschnitten aus einer Probe mit in der Datenbank gespeicherten Standards und zur entsprechenden Zuordnung bei Übereinstimmung, sowie (c) eine Anzeigeeinheit zum Anzeigen von zugeordneten Banden oder nicht zugeordneten Banden. Bevorzugt übermittelt der Detektor ein Signal an die angeschlossene Steuereinheit, beispielsweise einen Computer, mit dessen Hilfe ein digitalisierter Barcode der aufgetrennten Banden aufgrund ihrer unterschiedlichen Mobilität erstellt wird (wie beispielsweise das Ll-COR System beim automatischen Sequenzierer). Dabei ist bevorzugt der Detektor so ausgestaltet, daß parallele Bahnen von Banden gleichzeitig detektiert werden können. Dies erlaubt eine automatisierte Detektion von Banden aus Proben mit Banden von Standards bzw. Standardsets. Es müssen jedoch keine Standards gleichzeitig durch die Matrix migrieren, sondern es können Informationen zur Mobilität bereits in einer Datenbank gespeichert sein, wobei dann die vom Detektor ermittelten Daten mit denjenigen aus der Datenbank verglichen werden und somit eine Zuordnung der detektierten Banden zu bestimmten Mikroorganismen oder anderen Organismen erfolgen kann. Die erfindungsgemäße Anzeigeeinheit kann so eingestellt sein, daß sie je nach verwendeten Standardsets alle detektierten Banden, oder beispielsweise nur die pathogenen Organismen zugeordneten Banden anzeigt. Es kann auch ein Kollektor an das System angeschlossen sein, welcher sich zum Sammeln der Fraktionen von Nukleinsäureabschnitten eignet. Hierzu migrieren die Banden bis zum Ende der Auftrennungsmatrix und werden dann von einem Kollektorbehälter aufgefangen. Dieser kann beispielsweise aus einer Reihe von Gefäßen bestehen, welche beweglich am Ende der Matrix angebracht ist, so daß, wenn eine aus der Matrix aufgetretene Bande in einem Gefäß aufgefangen worden ist, ein leeres Gefäß an diese Stelle rücken kann.

Zusätzlich kann der Kollektor so mit der Steuereinheit verbunden sein, daß nach erfolgtem Detektieren und Vergleich der Probenbanden nur bei bestimmten identifizierten Banden ein Signal an den Kollektor zum Sammeln der in der Bande enthaltenen Nukleinsäureabschnitte weitergeleitet wird. Der Kollektor kann auch aus einer Absaugeeinrichtung bestehen.

Bevorzugt ist der Kollektor kompatibel mit marktüblichen PCR- und Sequenzierautomaten. Es kann sich bei den Kollektorgefäßen beispielsweise um eine Mikrotiterplatten oder um herkömmliche Reaktionsgefäße handeln.

Das erfindungsgemäße Verfahren und die erfindungsgemäße Vorrichtung sind für viele Arten zum Nachweis von Kontaminationen und der Diagnostik geeignet.

Unter Kontaminationen wird jede Art des Vorkommens von Organismen, insbesondere Mikroorganismen, verstanden, die in Probenmaterial vorkommen und mögliche Beeinträchtigungen oder Veränderungen hervorrufen können. Insbesondere ist hier die Lebensmittel- und Abwasseranalytik (z.B. die Reinigung von Biomatten in Klärwerken) zu nennen, oder der Nachweis von (Mikro-)Organismen auf Kunstobjekten und in Werkstoffen. Es können auch Parasiten, zum Beispiel Würmer und Einzeller, nachgewiesen werden.

Unter Diagnostik wird der Nachweis von Organismen, z.B. Mikrooganismen, verstanden. Insbesondere ist hier die Untersuchung von klinischen Proben und die Parasitologie zu nennen. Es können allerdings auch kriminalistische Untersuchungen vorgenommen werden. Außerdem zu nennen ist der Anwendungsbereich der Untersuchung von gentechnologisch veränderten Organismen, insbesondere solchen, die zu Lebensmitteln verarbeitet werden.

### Figurenbeschreibung:

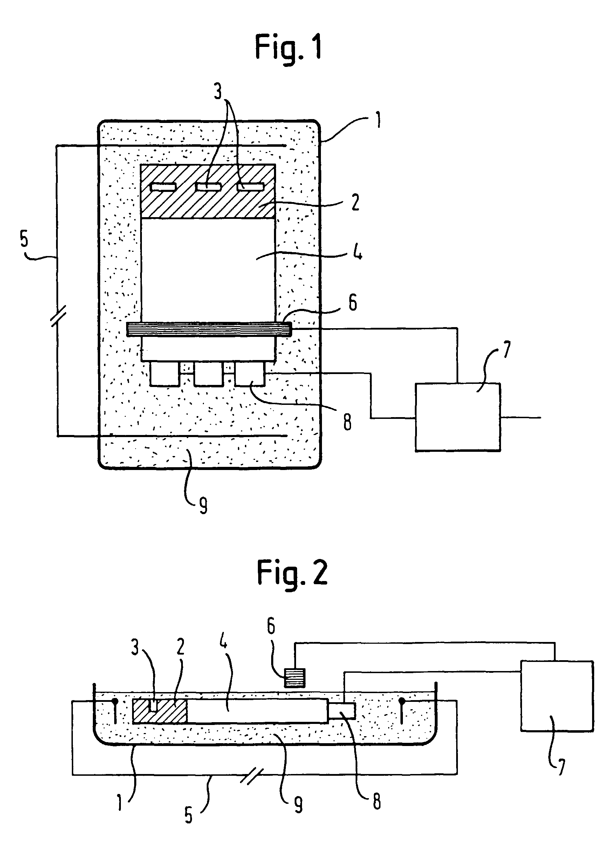

Figur 1: zeigt eine Draufsicht einer erfindungsgemässen Vorrichtung (schematisch) umfassend ein mit Elektrolysepuffer bedecktes Gel sowie einen Infrarot-Detektor und mehrere Kollektorbehälter, welche mit einer Steuereinheit verbunden sind.

Figur 2: zeigt eine Seitenansicht der Vorrichtung aus Figur 1.

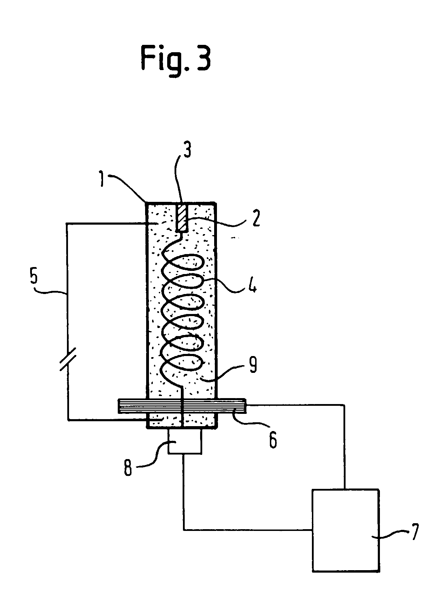

Figur 3: zeigt eine ähnliche Vorrichtung wie in Figur 1 und 2, außer daß die Matrix in Form einer Spule vorliegt.

### Beispiele

### Beispiel 1 Nachweis von Salmonellen in Lebensmitteln

In diesem Beispiel wurden 16S rDNA spezifische, bakterielle Primer verwendet, mit denen die 16S rDNA Fragmente aller auf der Hühnerhaut vorhandenen Bakterien amplifiziert wurden. Die Amplifikate wurden dann in einer DGGE-Analyse voneinander getrennt. Als Standard wurde die entsprechende für Salmonella spezifische Bande verwendet. Die Ergebnisse zeigen eindeutig das Vorhandensein der Salmonella-spezifischen Bande in der zu untersuchenden Probe, die damit eindeutig als Salmonella-positiv identifiziert werden konnte.

Durch Verwendung von Standards konnten Salmonella-positive Proben identifiziert werden.

### Beispiel 2 Nachweis unterschiedlicher pathogener Mikroorganismen in Lebensmitteln

In diesem Beispiel wurden dieselben Primer wie in Beispiel 1 verwendet und das Verfahren auf prinzipiell dieselbe Art und Weise durchgeführt. Bei Untersuchungen von Lebensmitteln mit dem erfindungsgemässen Verfahren und unter Verwendung eines Standards, der einen Satz Sequenzen von in Lebensmitteln häufig vorkommenden pathogenen Mikroorganismen enthält (Salmonellen, Listerien, Toxin produzierende E.colis), konnten kontaminierte und potentiell gesundheitsgefährdende Lebensmittel identifiziert werden.

### Beispiel 3 Nachweis von Lebensmittelverfälschungen

Untersucht wurde die Authentizität von Lebensmitteln, d.h. ob Billigmaterial zu hochwertigen Lebensmitteln beigemischt wurde. Durch Untersuchung von Rindfleischproben konnten unter Verwendung von Standards mit Sequenzen von verschiedenen für die Fleischproduktion verwendeten Tierarten (Rind, Schwein, Schaf, Pute, Huhn) verfälschte Lebensmittelproben identifiziert wurden, die nicht nur ein Signal für Rindfleisch, sondern auch für andere Fleischsorten, z.B. Putenfleisch, enthielten.

Geeignet waren solche Primer, die spezifisch sind für phylogenetische Markergene (z.B. ribosomale Gene, Cytochrom B, und andere)

### Beispiel 4 Nachweis von Parasiten

Säugetiere können durch eine Reihe von mehr oder weniger gesundheitsgefährdenden Parasiten befallen sein. Beispielsweise führt die Besiedelung der roten Blutkörperchen durch Protozoen häufig zu schweren Krankheiten und Todesfällen bei Mensch und Tier.

Durch eine Untersuchung solcher Blutproben mit organismusübergreifenden Primern können auf unterschiedlichen phylogenetischen Ebenen (z.B. alle Protozoen oder alle Piroplasmodien) alle potentiellen Krankheitserreger (wie z.B. Babesien) einer Organismengruppe in einem einzigen Nachweis identifiziert werden.

## Patentansprüche

1. Verfahren zum Nachweis von Organismen anhand ihrer Nukleinsäure, umfassend die Schritte:
(a) Bereitstellen einer Probe, die einen Organismus oder verschiedene Organismen oder genetisches Material davon enthalten kann,
(b) Organismusübergreifendes Erzeugen von Nukleinsäureabschnitten der Organismen, die eine sequenzspezifische Zuordnung zu den entsprechenden Organismen erlauben,
(c) Sequenzspezifisches Auftrennen der Nukleinsäureabschnitte mittels Migration durch eine Matrix,
(d) Automatisiertes Detektieren der aufgetrennten Nukleinsäureabschnitte während oder/und nach der Migration durch einen stationären oder mobilen Detektor,
(e) Mindestens teilweises Identifizieren von Organismen aufgrund der sequenzspezifischen Mobilität ihrer Nukleinsäureabschnitte durch Vergleich mit in einer Datenbank gespeicherten Sequenzen bekannter Organismen und der Mobilitätswerte dieser Sequenzen.

2. Verfahren nach Anspruch 1, dadurch gekennzeichnet, daß das Identifizieren in Schritt (e) durch eine Steuervorrichtung erfolgt, umfassend eine Datenbank, eine Vergleichs oder Zuordnungseinheit und eine Anzeigeeinheit, wobei die Mobilität der Nukleinsäureabschnitte mit in der Datenbank gespeicherten Werten für die Mobilität des oder der Standards verglichen werden, bei Übereinstimmung eine Zuordnung der Nukleinsäureabschnitte zu bekannten Organismen erfolgt.

3. Verfahren nach Anspruch 1, dadurch gekennzeichnet, daß das Identifizieren von Organismen in Schritt (e) dadurch erfolgt, daß parallel zur Probe mit den zu identifizierenden Organismen ein Standard mit Nukleinsäureabschnitten bekannter Organismen durch die Matrix migriert, so daß durch einen Vergleich des Migrationsverhaltens der in dem Standard enthaltenen Nukleinsäureabschnitte mit denen der Probe eine Identifizierung der Organismen in der Probe erfolgen kann.

4. Verfahren nach einem der vorhergehenden Ansprüche, dadurch gekennzeichnet, daß das Sammeln von Nukleinsäureabschnitten durchgeführt wird, indem die Migration durch die Matrix solange durchgeführt wird, bis die aufgetrennten Nukleinsäureabschnitte aus der Matrix austreten

5. Vorrichtung umfassend eine Matrix zum sequenzspezifischen Auftrennen in räumlich voneinander getrennte Banden von Nukleinsäureabschnitten mit gleicher oder annähernd gleicher Länge, wobei das Auftrennen mittels Migration der Nukleinsäureabschnitte vorzugsweise vollständig durch die Matrix hindurch geschieht.

6. Vorrichtung umfassend einen Detektor zum Erfassen der aufgetrennten Nukleinsäureabschnitte während und/oder nach der Migration durch die Matrix.

7. Vorrichtung umfassend einen Kollektor, der einen oder mehrere beweglich angeordnete Auffangbehälter umfaßt, die das Auffangen von einzelnen Banden während und/oder nach der Migration ermöglichen.

8. Vorrichtung umfassend eine Datenbank zum Speichern von Werten für die Mobilität von detektierten Nukleinsäureabschnitten, eine Vergleichs- und Zuordnungseinheit zum Vergleich der Mobilität von Nukleinsäureabschnitten aus einer Probe mit in der Datenbank gespeicherten Standards und zur entsprechenden Zuordnung bei Übereinstimmung, sowie eine Anzeigeeinheit zum Anzeigen von zugeordneten Organismen.

9. Anwendung des Verfahrens nach einem oder mehrerer der Ansprüche 1 bis 4, zum Nachweis von Kontaminationen mit einem oder mehrer der Vorrichtungsansprüche 5 bis 8.

10. Anwendung des Verfahrens nach einem oder mehrerer der Ansprüche 1 bis 4, zur Diagnostik von Organismen mit einem oder mehrer der Vorrichtungsansprüche 5 bis 8.
